# EUROPEAN PATENT SPECIFICATION

(11) **EP 1 487 837 B1**
(45) Date of publication and mention of the grant of the patent: **30.08.2006**
(21) Application number: 02717017.4
(22) Date of filing: 25.03.2002
(51) Int. Cl.: C07D 487/04

(54) **AN IMPROVED PROCESS FOR THE PRODUCTION OF VASICINE**
VERBESSERTES VERFAHREN ZUR HERSTELLUNG VON VASICINE
PROCEDE AMELIORE DE PRODUCTION DE VASICINE

(43) Date of publication of application: 22.12.2004
(73) Proprietor: COUNCIL OF SCIENTIFIC AND INDUSTRIAL RESEARCH, New Delhi 100 001 (IN)
(72) Inventor: CHATTOPADHYAY, Sunil Kumar, Lucknow, 226 015 Uttar Pradesh (IN); BAGCHI, Guru Das, Lucknow, 226 015 Uttar Pradesh (IN); DWIVEDI, Prem Dutt, Lucknow, 226 015 Uttar Pradesh (IN); SRIVASTAVA, Sachin, Lucknow, 226 015 Uttar Pradesh (IN)
(74) Representative: Frith, Richard William
(86) International application number: PCT/IB2002/001209
(87) International publication number: WO 2003/080618

(56) References cited:
- SPÄTH, E.; KESZTLER, F.: "Über das l-Peganin (l-Vasicin) aus Adhatoda Vasica Nees" CHEM. BER., vol. 69, 1936, pages 384-386, XP001097937
- MEHTA D R ET AL: "VASICINONE. A BRONCHODILATOR PRINCIPLE FROM ADHATODA VASICA NEES (N.O. ACANTHACEAE)" JOURNAL OF ORGANIC CHEMISTRY, AMERICAN CHEMICAL SOCIETY. EASTON, US, vol. 28, 1 February 1963 (1963-02-01), pages 445-448, XP000605375 ISSN: 0022-3263 cited in the application

## Description

This invention relates to an improved process for the production of vasicine. More particularly, this invention relates to a process for the production of a biologically active compound vasicine of formula (1) from the leaves of *Adhatoda vasica*. 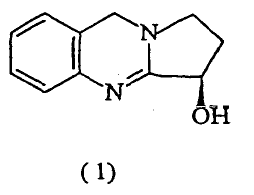 *Adhatoda vasica* belonging to the family Acanthaceae is commonly known as 'Arusa', 'Vasaka' or 'Malabarnut'. It is an evergreen and perennial shrub and attains a height up to2.0m at north Indian plain conditions. The plant is a well-known drug of Ayurvedic system of medicine. In India, it has been used for over 2000 years for the treatment of respiratory complaints and diseases like coughing, asthma and colds. The most studied chemical component of the plant is vasicine. It showed bronchodialatory activity both in vitro and in vivo comparable to theophylline. It was also observed that vasicine initiated rhythmic contractions of human myometrial strips from both non-pregnant and pregnant uteri. The effect was comparable with that of oxytocin and methergin (C.K.Atal. Chemistry and Pharmacology of vasicine- a new oxytocic and abortifacient. Raj. Bandhu Ind. Co., New Delhi, 1980).
According to a prior art process vasicine can be isolated from the leaves of *A.vasica* by extracting the leaves of the plant with 95% alcohol, treating the concentrated alcoholic extract with aqueous 2% H₂SO₄, basifying the aqueous acidic solution with ammonia and extracting with chloroform, concentrating the chloroform gave a extract which was again dissolved in aqueous 2% H₂SO₄ and repeating the process of basification with ammonia, followed by extraction with chloroform (C.K.Atal. Chemistry and Pharmacology of vasicine- a new oxytocic and abortifacient. Raj. Bandhu Ind. Co., New Delhi, 1980). The drawback of the process include use of strong mineral acid like H₂SO₄ for extraction which result in considerable degradation of vasicine, which is further aggravated by repeating the process of same mineral acid treatment twice.

In another prior art, process vasicine was isolated from *A. vasica* leaves as follows (D.R.Mehta, J.S.Naravane and R.M.Desair. J. Org. chem. 28, 445-448, 1963). The leaves were refluxed with 90% alcohol and after evaporation of the solvent the alcohol extract thus obtained was extracted with hot distilled water and the aqueous extract was filtered. The filtrate was extracted with chloroform to remove the coloring matters and then made alkaline with 5% caustic soda, and again extracted with chloroform. The combined chloroform extracts were extracted with 5% hydrochloric acid, then acidic solution was made alkaline with ammonia and again extracted with chloroform.
After repeating the process twice the final chloroform extract was concentrated to give a crude total alkaloid from which vasicine was isolated as vasicine hydrochloride yield 2g.
The first drawback of the above process includes the extraction of the alcohol extract with hot water, which has two drawbacks- viz, (a) vasicine could not be quantitatively extracted from its aqueous solution and (b) hot water extraction will convert vasicine into its auto oxidation product vasicinone.
Second drawback of the process is the use of 5% mineral acid like hydrochloric acid for its extraction and that also, twice. The use of a strong mineral acid degrades vasicine considerably and thus results in a lower yield of vasicine.
Späth, E; Keztler, F. Chem. Ber. 1936, 384-386 discloses a process for isolating vasicine from Adhatoda vasica leaves wherein alkaloids are precipitated from an ethereal solution, the precipitate isolated and vasicine obtained by further extraction and recrystallisation steps.
The object of the present invention is to develop an improved process for isolation of vasicine of formula (1) from the leaves of the plant *A. vasica* with high yields.
Another object of the present invention is to develop a processing technology for its isolation, which does not use any chromatographic separation for its isolation.
Still another object of the present invention is to develop a processing technology for isolation of vasicine, which can be applicable to commercial scale production of this important molecule.
Accordingly, in order to overcome the drawbacks of the prior art processes, the applicants have developed a simple and practical process adaptable to commercial production of vasicine. The process comprises, amongst other things, (a) extracting air dried, pulverized leaves of the *Adhatoda vasica* with alcohol at room temperature, evaporating the solvent to obtain an alcoholic extract, (b) stirring the resultant residue with aqueous solution of an organic acid for 2-24 hours and (c) extracting the acidic layer with organic solvent, (d) basifying the aqueous acidic solution with a suitable base and extracting the basic layer with organic solvent exhaustively and concentrating the organic phase gave a semi solid residue of vasicine, (d) isolating vasicine from the resultant residue by treating it with a suitable solvent or mixture of solvents and filtering.

Accordingly, the present invention provides an improved process for the production of vasicine of formula (1) from the *Adhatoda vasica*, wherein no chromatographic separation is used said process comprising the steps of: 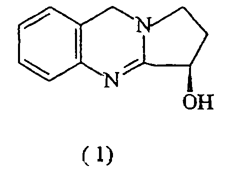
a. extracting the dried and pulverized leaves with an alcoholic extract at an ambient temperature,
b. concentrating the alcoholic extract of step (a) to obtain a concentrated extract,
c. treating and stirring extract of step (b) with an aqueous organic acid for 2-24 hours.
d. extracting the acid solution of steps (c) with an organic solvent,
e. separating the organic layer and aqueous acidic layer of step (d),
f. basifying the aqueous acidic solution of step (e) with a base,
g. extracting the basified solution of step (f) with an organic solvent,
h. separating the organic layer of step (g) drying and filtering,
i. evaporating the organic layer of step (h) to obtain an amorphous residue, and
j. treating the amorphous residue of step (i) with a mixture of petroleum ether-acetone in the ratio of 1:1 to 2:1 to obtain vasicine that has a minimum parity of 80%.
In an embodiment of the invention, wherein in step (a and b) the alcohol used is selected from a group consisting of methanol, ethanol, propanol, n-butanol and preferably methanol and ethanol.
Still another embodiment of the invention, wherein in step (c) the organic acid used is selected from group consisting of citric acid, oxalic acid, tartaric acid, acetic acid or propionic acid.
Still another embodiment of the invention relates to the use of organic acid in step (c), which is preferably citric acid.

Yet another embodiment of the invention, the organic solvent used in step (d) is selected from the group consisting of chloroform, dichloromethane, ether, ethyl acetate, toluene and more preferably chloroform and most preferably dichloromethane.

Yet another embodiment of the invention, the base used in step (f) is selected from a group consisting of aqueous ammonia, sodium hydroxide, sodium carbonate, potassium carbonate, lithium hydroxide and preferably aqueous ammonia.

Yet another embodiment of the invention, the organic solvent used in step (g) is selected from a group consisting chloroform, dichloromethane, ether, ethyl acetate, toluene more preferably dichloromethane and most preferably chloroform.

Yet another embodiment of the invention, the recovery of vasicine obtained by this process is 2.0%, which is not obtained so far by any methods reported.

Yet another embodiment the vasicine is quantitatively extracted from the raw material.

Yet, another embodiment of the invention relates to a process, wherein the said process can be applied for isolation of vasicine irrespective of plant species.

In yet another embodiment of the invention depending upon the source of the plant material, the purity of the vasicine is affected.

The invention is described in detail in the examples given below which are provided to illustrate the invention and therefore should not be construed to limit the scope of the invention.

### Example I

Air-dried, powdered leaf of *A.vasica* (1Kg.) was extracted with methanol (3 litres x 5) at 20-40°C for seventy-two hours. The combined methanol extract was concentrated under reduced pressure to give a concentrated extract (200 ml). The said extract was then treated with a aqueous solution of citric acid (1.0 litre) and was stirred at ambient temperature for 2-24 hours. It was filtered and the clear solution thus obtained was extracted with chloroform (1.0 litre x 3). The aqueous acidic layer was then basified with ammonia solution to pH 9.5 and the basic aqueous solution was extracted with chloroform (1 litre x 3) and the chloroform extract was concentrated under reduced pressure to give an amorphous residue of vasicine; the amorphous residue thus obtained was triturated with a mixture of acetone-petroleum ether (Ratio of pet.ether:acetone 1:1; 100 ml) with stirring and filtered to give vasicine (20gms) with 80% purity

### Example II

An air-dried, powdered leaf of A.vasica (1Kg) was extracted with ethanol (3 litre x 5) under the same condition described above to give a concentrated extract (200 ml). The said extract was then treated with a aqueous solution of tartaric acid (1.0 litre) and was stirred at ambient temperature for 2-24 hours. The acidic solution after filtration was extracted with dichloromethane (1.0 litre x 3); then the said acidic solution was basified with aqueous solution of sodium carbonate and was extracted with dichloromethane (1 litre x 3). The dichloromethane extract after concentration gave amorphous vasicine, which was then treated with ether -petroleum ether mixture in the ratio of petroleum ether: acetone 2:1, with stirring and filtered to give vasicine (20 g) with 84% purity

### Advantages:

(1) No chromatographic separation is needed at any stage for isolation of vasicine. Thus, the process described is cost effective and adaptable for large-scale production of vasicine.
(2) The process described for isolation of vasicine involves normal condition of temperature and pressure. Thus, the process is simple, straightforward and adaptable to commercial production.
(3) The recovery of vasicine obtained by this process is 2.0%, which is not obtained so far by any methods reported.
(4) The process can be applied for isolation of vasicine irrespective of plant species. Thus the process described here, is a general process for isolation of vasicine.

## Claims

1. An improved process for the production of vasicine of formula (1) from the *Adhatoda vasica*, wherein no chromatographic separation is used, said process comprising the steps of: 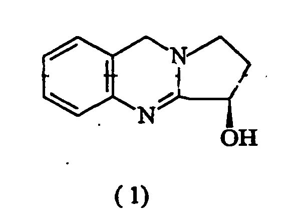
a. extracting the dried and pulverized leaves with an alcoholic extract at an ambient temperature,
b. concentrating the alcoholic extract of step (a) to obtain a concentrated extract,
c. treating and stirring extract of step (b) with an aqueous organic acid for 2-24 hours.
d. extracting the acid solution of steps (c) with an organic solvent,
e. separating the organic layer and aqueous acidic layer of step (d),
f. basifying the aqueous acidic solution of step (e) with a base,
g. extracting the basified solution of step (f) with an organic solvent,
h. separating the organic layer of step (g) drying and filtering,
i. evaporating the organic layer of step (h) to obtain an amorphous residue, and
j. treating the amorphous residue of step (i) with a mixture of petroleum ether-acetone in the ratio of 1:1 to 2:1 to obtain vasicine that has minimum purity of 80%.

2. A process as claimed in claim 1, wherein in step (a) the alcohol used is selected from a group consisting of methanol, ethanol, propanol, n-butanol and preferably methanol and ethanol.

3. A process as claimed in claim 1, wherein in step (b) the organic acid used is selected from group consisting of citric acid, oxalic acid, tartaric acid, acetic acid or propionic acid.

4. A process as claimed in claim 1, wherein in step (b) the preferred organic acid is citric acid

5. A process as claimed in claim 1, wherein in step (d) the organic solvent used is selected from the group consisting of chloroform, dichloromethane, ether, ethyl acetate, toluene and more preferably chloroform and most preferably dichloromethane.

6. A process as claimed in claim 1, wherein in step (f) the base used is selected from a group consisting of aqueous ammonia, sodium hydroxide, sodium carbonate, potassium carbonate, lithium hydroxide and preferably aqueous ammonia.

7. A process as claimed in claim 1, wherein in step (g) the organic solvent used is selected from a group consisting of chloroform, dichloromethane, ether, ethyl acetate, toluene more preferably dichloromethane and most preferably chloroform.

8. A process as claimed in claim 1, wherein the recovery of vasicine obtained is 2.0% based on the weight of Adhatoda vasica.

## Patentansprüche

1. Verbessertes Verfahren für die Produktion von Vasicin der Formel (1) aus *Adhatoda vasica*, bei dem keine chromatographische Trennung verwendet wird, wobei das Verfahren die folgenden Schritte umfaßt: 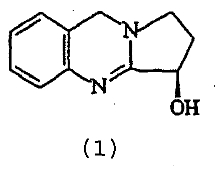
a. Extrahieren der getrockneten und pulverisierten Blätter mit einem alkoholischen Extrakt bei Raumtemperatur,
b. Konzentrieren des alkoholischen Extrakts von Schritt (a), wodurch man einen konzentrierten Extrakt erhält,
c. 2-24 stündiges Behandeln und Rühren des Extrakts aus Schritt (b) mit einer wäßrigen organischen Säure,
d. Extrahieren der sauren Lösung von Schritt (c) mit einem organischen Lösungsmittel,
e. Trennen der organischen Schicht und der wäßrig-sauren Schicht aus Schritt (d),
f. Basischstellen der wäßrig-sauren Lösung von Schritt (e) mit einer Base,
g. Extrahieren der basisch gestellten Lösung aus Schritt (f) mit einem organischen Lösungsmittel,
h. Abtrennen der organischen Schicht aus Schritt (g), Trocknen und Filtrieren,
i. Eindampfen der organischen Schicht aus Schritt (h), wodurch man zu einem amorphen Rückstand gelangt, und
j. Behandeln des amorphen Rückstands aus Schritt (i) mit einer Mischung aus Petrolether und Aceton im Verhältnis 1:1 bis 2:1, wodurch man zu Vasicin mit einer Reinheit von mindestens 80% gelangt.

2. Verfahren nach Anspruch 1, wobei in Schritt (a) der verwendete Alkohol aus einer Reihe bestehend aus Methanol, Ethanol, Propanol, n-Butanol und vorzugsweise Methanol und Ethanol stammt.

3. Verfahren nach Anspruch 1, wobei in Schritt (b) die verwendete organische Säure aus der Reihe bestehend aus Citronensäure, Oxalsäure, Weinsäure, Essigsäure oder Propionsäure stammt.

4. Verfahren nach Anspruch 1, wobei es sich in Schritt (b) bei der bevorzugten organischen Säure um Citronensäure handelt.

5. Verfahren nach Anspruch 1, wobei in Schritt (d) das verwendete organische Lösungsmittel aus der Reihe bestehend aus Chloroform, Dichlormethan, Ether, Essigester, Toluol, stärker bevorzugt Chloroform, am stärksten bevorzugt Dichlormethan stammt.

6. Verfahren nach Anspruch 1, wobei in Schritt (f) die verwendete Base aus einer Reihe bestehend aus wäßrigem Ammoniak, Natriumhydroxid, Natriumcarbonat, Kaliumcarbonat, Lithiumhydroxid und vorzugsweise wäßrigem Ammoniak stammt.

7. Verfahren nach Anspruch 1, wobei in Schritt (g) das verwendete organische Lösungsmittel aus der Reihe bestehend aus Chloroform, Dichlormethan, Ether, Essigester, Toluol, stärker bevorzugt Dichlormethan, am stärksten bevorzugt Chloroform stammt.

8. Verfahren nach Anspruch 1, wobei die erhaltene Vasicinausbeute 2,0% in bezug auf das *Adhatodavasica*-Gewicht ausmacht.

## Revendications

1. Procédé amélioré de production de vasicine de formule (1) à partir d'*Adhatoda vasica*, **caractérisé en ce qu'**aucune séparation chromatographique n'est utilisée, ledit procédé comprenant les étapes consistant à: 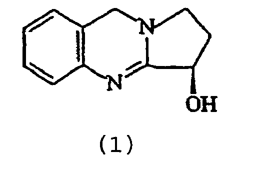
a. extraire les feuilles séchées et pulvérisées avec un extrait alcoolique à température ambiante,
b. concentrer l'extrait alcoolique de l'étape (a) pour obtenir un extrait concentré,
c. traiter et agiter l'extrait de l'étape (b) avec un acide organique aqueux pendant 2 à 24 heures.
d. extraire la solution acide de l'étape(c) avec un solvant organique,
e. séparer la couche organique et la couche acide aqueuse de l'étape (d),
f. basifier la solution acide aqueuse de l'étape (e) avec une base,
g. extraire la solution basifiée de l'étape (f) avec un solvant organique,
h. séparer la couche organique de l'étape (g), la sécher et la filtrer,
i. évaporer la couche organique de l'étape (h) pour obtenir un résidu amorphe, et
j. traiter le résidu amorphe de l'étape (i) avec un mélange éther de pétrole-acétone dans un rapport de 1:1 à 2:1, pour obtenir de la vasicine ayant une pureté minimale de 80%.

2. Procédé selon la revendication 1, **caractérisé en ce que** dans l'étape (a), l'alcool utilisé est choisi dans un groupe constitué du méthanol, de l'éthanol, du propanol, du n-butanol et de préférence du méthanol et de l'éthanol.

3. Procédé selon la revendication 1, **caractérisé en ce que** dans l'étape (b), l'acide organique utilisé est choisi dans le groupe constitué de l'acide citrique, de l'acide oxalique, de l'acide tartrique, de l'acide acétique et de l'acide propionique.

4. Procédé selon la revendication 1, **caractérisé en ce que** dans l'étape (b), l'acide organique préféré est l'acide citrique.

5. Procédé selon la revendication 1, **caractérisé en ce que** dans l'étape (d), le solvant organique utilisé est choisi dans le groupe constitué du chloroforme, du dichlorométhane, de l'éther, de l'acétate d'éthyle, du toluène et est plus préférablement le chloroforme et de façon tout à fait préférée le dichlorométhane.

6. Procédé selon la revendication 1, **caractérisé en ce que** dans l'étape (f), la base utilisée est choisie dans un groupe constitué de l'ammoniaque, de l'hydroxyde de sodium, du carbonate de sodium, du carbonate de potassium, de l'hydroxyde de lithium et est de préférence l'ammoniaque.

7. Procédé selon la revendication 1, **caractérisé en ce que** dans l'étape (g), le solvant organique utilisé est choisi dans le groupe constitué du chloroforme, du dichlorométhane, de l'éther, de l'acétate d'éthyle, du toluène et est plus préférablement le dichlorométhane et de façon tout à fait préférée le chloroforme.

8. Procédé selon la revendication 1, **caractérisé en ce que** la récupération de vasicine obtenue représente 2,0% sur la base du poids d'*Adhatoda vasica*.
